# EUROPÄISCHE PATENTSCHRIFT

(11) **EP 3 246 032 B1**
(45) Veröffentlichungstag und Bekanntmachung des Hinweises auf die Patenterteilung: **11.08.2021**
(21) Anmeldenummer: 17171880.2
(22) Anmeldetag: 19.05.2017
(51) Int. Cl.: A61K 31/593, A61K 31/663, A61K 33/06, A61P 19/08, A61P 19/10, A61K 31/675, A61P 3/02, A61P 9/06, A61P 9/10, A61P 19/00, A61P 29/00, A61P 35/00

(54) **PHARMAZEUTISCHE ZUSAMMENSETZUNGEN MIT ZOLEDRONSAEURE, KALZIUM UND VITAMIN D, GEEIGNET ZUR BEHANDLUNG UND/ODER PROPHYLAXE VON ERKRANKUNGEN DES KNOCHENSTOFFWECHSELS UND VON THERAPIEBEDINGTEN NEBENWIRKUNGEN WIE HYPOKALZAEMIEN**
PHARMACEUTICAL COMPOSITIONS COMPRISING ZOLEDRONIC ACID, CALCIUM AND VITAMIN D, SUITABLE FOR THE TREATMENT AND/OR PROPHYLAXIS OF DISEASES RELATED TO BONE METABOLISM AND OF TREATMENT-RELATED SIDE EFFECTS SUCH AS HYPOCALCAEMIA
COMPOSITIONS PHARMACEUTIQUES COMPRENANT DE L'ACIDE ZÉLODRONIQUE, DU CALCIUM ET DE LA VITAMINE D, CONVENANT AU TRAITEMENT ET/OU À LA PROPHYLAXIE DE MALADIES LIÉES AU MÉTABOLISME OSSEUX ET DES EFFETS SECONDAIRES DES THÉRAPIES COMME L'HYPOCALCÉMIE

(30) Priorität: 20.05.2016 DE 102016006446
(43) Veröffentlichungstag der Anmeldung: 22.11.2017
(73) Patentinhaber: Karl, Christoph, 8274 Tägerwilen (CH)
(72) Erfinder: Thierolf, Rüdiger, 64832 Babenhausen (DE); Karl, Christoph, 8274 Tägerwilen (CH)
(74) Vertreter: Boergen, Xenia

(56) Entgegenhaltungen:
- WO-A1-2004/024166
- WO-A1-2008/116133
- WO-A2-2016/020508
- Anonymous: "Zometa - FDA prescribing information, side effects and uses", , 14. Mai 2016 (2016-05-14), XP055399226, Gefunden im Internet: URL:https://web.archive.org/web/2016051408 5901/https://www.drugs.com/pro/zometa.html [gefunden am 2017-08-17]
- BOURKE S ET AL: "The impact of dietary calcium intake and vitamin D status on the effects of zoledronate", OSTEOPOROSIS INTERNATIONAL ; WITH OTHER METABOLIC BONE DISEASES, SPRINGER-VERLAG, LO, Bd. 24, Nr. 1, 15. August 2012 (2012-08-15), Seiten 349-354, XP035158420, ISSN: 1433-2965, DOI: 10.1007/S00198-012-2117-4

## Beschreibung

### Technisches Gebiet

Die vorliegende Erfindung beinhaltet pharmazeutische Zusammensetzungen, umfassend Zoledronsäure, Kalzium und Vitamin D, die zur Behandlung und/oder Prophylaxe von therapiebedingten Nebenwirkungen wie Hypokalzämien bei Erkrankungen des Knochenstoffwechsels verursacht durch die Wirkung der Zoledronsäure, geeignet sind.

### Beschreibung

Die Behandlung von Erkrankungen des Knochenstoffwechsels wie der Osteoporose, Tumorerkrankungen inklusive induziertem Knochenverlust (CTIBL, cancer treatmentinduced bone loss), Morbus Paget, oder der Hypokalzämie bilden einen bedeutenden Schwerpunkt in der medizinischen Forschung. Der menschliche Knochen besteht aus einer organischen Matrix, die mit anorganischen Salzen - vorwiegend Kalzium und Phosphat in Form von Hydroxylapatit (Ca₃(PO₄)₂ • Ca(OH)₂) kombiniert ist. Als Grundprinzip liegt der Knochenphysiologie beim Menschen ein ständiger Auf- und Abbau (bone turnover) von Knochensubstanz zugrunde. Knochen AUF- und AB-Bau im Rahmen des fortwährenden Remodellings ist dabei hormonell (im Wesentlichen durch Östrogen, Parathormon (PTH)) und durch Signaltransduktionswege (im Wesentlichen RANKL ("receptor activator of nuclear factor-kB ligand")/OPG (Osteoprotegerin) und Ca/PTH/Vitamin D3) reguliert. Während in der Kindheit und v.a. der Pubertät mehr Knochen auf- als abgebaut wird, kommt es im Alter, bei Frauen insbesondere während und nach der Menopause, zunehmend zum 'netto'-Knochenabbau.

Im Laufe des menschlichen Lebens ist Knochen fortwährend physiologischen mechanischen Belastungen ausgesetzt und passt sich entsprechend an. Erstmals beschrieben wurde dies von Julius Wolff 1862 und das Wolffsche Gesetz stellt die Grundlage für das biomechanische Verständnis des Knochens dar. Wolff konnte bei der Untersuchung von Femurköpfen zeigen, dass der Knochen sich in seiner Form an die Funktion anpasst und bei dauerhafter Entlastung degeneriert. So wird im Rahmen des "Remodelling" nicht belasteter Knochen abgebaut, was in den 'Bed-rest'-Studien mit absoluter Bettruhe über einen Zeitraum von 60 Tagen untersucht wurde. Mechanisch stark belasteter Knochen wird hingegen entsprechend der Kraftlinien verstärkt, was beispielhaft an Unterarmknochen von Tennisspielern gezeigt werden konnte.

Neben regelmäßigen Umbauvorgängen kommt es bei Krafteinwirkung auf den Knochen zu Mikroschäden oder Kontinuitätsunterbrechungen (Frakturen) mit und ohne Defektbildung, die narbenlos ausheilen können. Bei anorganischen Materialien führen repetitive Belastungen unterhalb der Bruchgrenze zur Materialermüdung/zum Materialermüdungsbruch, indem kleine Risse entstehen und wachsen, bis sie eine kritische Größe erreichen und das Material bricht. Als quasi-sprödes Material, das durch Ausbildung von kleinen Rissen (sogenannte "micro-cracks") Energie absorbiert, kann es Knochenfrakturen vermeiden. "Micro-cracks" wurden bereits 1960 von Harold Frost beschrieben und sind klar begrenzte Risse von 50-100µm Länge, die vornehmlich im interstitiellen Knochen auftreten. Sie sind in der Regel entsprechend der biomechanischen Belastung in der longitudinalen Achse länger als in der transversalen Achse, entstehen bereits bei physiologischen, repetitiven Belastungen wie Gehen und Laufen im trabekulären wie kortikalen Knochen und treten signifikant vermehrt im Alter auf.

Im Knochen bleiben "micro-cracks" in der Regel klinisch inapparent, da sie fortwährend im Rahmen des "Remodelling" repariert und geheilt werden. Durch den Riss kommt es zur Apoptose von Osteozyten, welche Faktoren freisetzen, u.a. RANKL. Diese stoßen die osteoklastäre Resorption an, mit konsekutivem osteoblastärem Knochenanbau.

Während gesunder Knochen das Fortschreiten von Mikroschäden verhindert und repariert, kann es im gealterten Knochen oder unter z.B. Suppression des "Remodellings" durch hoch potente antiresorptive Medikamente wie beispielsweise Bisphosphonate oder Anti-RANKL-Antikörpern zur Akkumulation und gegebenenfalls erhöhter Bruchanfälligkeit kommen, z.B. bei atypischen Femurfrakturen.

Bei den fortwährend im Rahmen des Remodellings stattfindenden Umbauvorgängen am Knochen sind im Wesentlichen Osteoblasten, Osteoklasten und Osteozyten involviert. Während Osteoklasten den Knochen abbauen, können Osteoblasten den Knochen anbauen.

Die Rolle der Osteozyten ist letztlich bis heute noch nicht bis ins Detail geklärt. Es handelt sich dabei um im Knochen eingemauerte terminal differenzierte Zellen osteoblastären Ursprungs, welche untereinander verbunden sind und kommunizieren. Kommt es zu Verletzungen der Osteozyten-Ausläufer, z.B. im Rahmen der oben beschriebenen Microcracks, wird zunächst die osteoklastäre Resorption initiiert, auf welche dann wiederum der osteoblastäre Knochenanbau folgt. Die Interaktion zwischen Osteoblasten, Osteoklasten und Osteozyten ist im Wesentlichen durch den RANKL/OPG-Signaltransduktionsweg (Differenzierung von Osteoklasten) und den WNT/DKK/SOST (WNT ist ein Signalprotein, welches sich aus dem Wingless (Wg) und Int-1-Protein zusammensetzt; DKK=Dickkopf; SOST=Symbol für das Protein Sclerostin))-Pathway (Differenzierung der Osteoblasten) gesteuert.

Beim Knochenanbau sind vor allem der WNT-Pathway zur Differenzierung der Osteoblasten als auch der PTH-Signaltransduktionsweg involviert. Die Differenzierung von hämatopoetischen Stammzellen zu Osteoklasten wird über den RANKL/OPG-Signaltransduktionsweg gesteuert. Die Osteoklasten werden durch Zoledronsäure inhibiert mit der Folge, dass kein Kalzium aus dem Knochen freigesetzt wird.

Der Knochen dient als Kalziumspeicher für den menschlichen Körper und ist somit entscheidend an der Kalzium-Phosphat-Homöostase beteiligt. Durch Knochenauf und - abbau können überschüssige Kalziumreserven aus dem Blut gespeichert oder auch im Bedarfsfall wieder zur Verfügung gestellt werden.

Bei diversen Erkrankungen des Knochenstoffwechsels ist dieses empfindliche Gleichgewicht gestört. Die häufigste Erkrankung des Knochenstoffwechsels ist die Osteoporose. Erkrankungen, welche den Knochenstoffwechsel beeinflussen, sind ferner Morbus Paget, Osteogenesis imperfecta, primäre Knochentumore wie das multiple Myelom, Plasmozytom, Osteosarkom und Knochenmetastasen. Bei Komplikationen durch Knochenmetastasen/Knochentumore spricht man im englischen Sprachgebrauch von "skeletal related events" (SRE oder "skelettbezogene Komplikationen"). Der Begriff "skelettbezogene Komplikationen" (SRE) beinhaltet akute Ereignisse wie pathologische Knochenfrakturen, Rückenmarkskompression, Knochenschmerzen oder eine tumorinduzierte Hyperkalzämie sowie therapeutische Interventionen wie Knochenbestrahlung oder operative Eingriffe am Knochen, die zum Beispiel bei Patienten mit Knochenmetastasen oder primären Knochentumoren auftreten können.

Osteoporose bewirkt eine Abnahme der Knochendichte, die zusammen mit der verringerten Knochenqualität zu einem vermehrten Knochenbruchrisiko führt. Als Osteoporose wird derzeit eine messbare Knochendichteminderung kleiner -2,5 Standardabweichungen (sogenannter T-Wert oder englisch T-score) von einem gesunden Kollektiv (Frauen, 30 Jahre), gemessen mittels DXA (dual-energy X-ray absorptiometry) von der WHO definiert. Durch einen stattgefundenen Knochenbruch spricht man von einer manifesten Osteoporose. Osteoporose ist somit eine systemische Erkrankung des Knochens, die sich häufig durch eine Fraktur bei inadäquatem Trauma manifestiert. In Deutschland sind ca. 6 Millionen Menschen von der Volkskrankheit Osteoporose betroffen und jährlich erleiden Osteoporosepatienten mehr als 720.000 Frakturen. Die postmenopausale Osteoporose ist die weitaus häufigste Ursache bei vertebralen und nicht-vertebralen Fragilitätsfrakturen. Das Risiko eine Fraktur zu erleiden steigt mit zunehmendem Alter erheblich an.

Bereits in den ersten 5 Jahren nach der Menopause wird mit ausbleibender Östrogenproduktion ein individuell unterschiedlich starker Abbau der Knochenmasse beobachtet. Dabei kann der postmenopausale Knochenmasseverlust bis zu 15% pro Jahr betragen und führt bei Frauen mit geringer Spitzenknochenmasse sehr schnell zur ersten osteoporotischen Fraktur.

Mangelhafte Kalziumzufuhr sowie ein dezimierter Vitamin D-Spiegel im Blut führen in der Bilanz zu einem Kalziumabbau im Knochen. Es ist außerdem zu erwarten, dass mit zunehmendem Alter weitere Pathomechanismen hinzukommen, die direkt oder indirekt den Knochenabbau fördern. Bis zum 65. Lebensjahr hat sich zum Beispiel die Fähigkeit Kalziumionen aus der Nahrung aufzunehmen um etwa 50% reduziert (im Vergleich zu Jugendlichen). Altersbedingte Begleiterkrankungen, wie Diabetes mellitus, eingeschränkte Nierenfunktion, Immobilität oder eine Therapie mit Glitazonen, sind nur einige Beispiele aus der langen Liste an Risikofaktoren, die nachweislich einen additiven Effekt auf den Knochenabbau haben und damit ein latentes Fortschreiten der Osteoporose fördern.

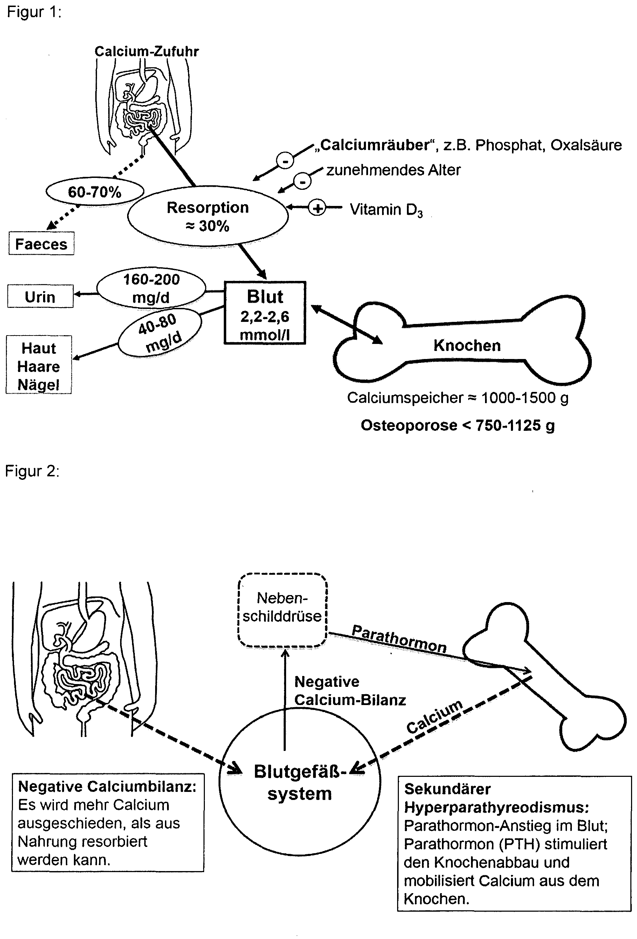

Die Figur 1 verdeutlicht, dass etwa nur ein Drittel der im Verdauungstrakt angebotenen Kalziummenge resorbiert wird. 65-70% des Nahrungskalziums wird mit dem Stuhl wieder ausgeschieden. Der Resorptionsvorgang selbst kann zusätzlich durch verschiedene Faktoren positiv oder negativ beeinflusst werden. Zum Beispiel Nahrungsmittel, die sehr stark mit Phosphaten angereichert sind, können die Aufnahme von Kalzium extrem behindern.

Zu den Hormonen, die die Kalzium-Homöostase steuern, zählen neben den Sexualhormonen (Östrogen, Testosteron) v.a. Calcitonin, Parathormon (Parathyrin) und Calcitriol (aktive Form von Vitamin D3). Calcitriol ist essentiell, um die Absorption von Kalzium und Phosphat im Dünndarm zu ermöglichen. Außerdem steigert Calcitriol die Rückresorption von Kalzium in den Nieren und stimuliert die Knochenmineralisation (Einbau von Kalzium in die Knochenmatrix).

Beispielsweise entsteht eine negative Kalziumbilanz, wenn der Körper mehr Kalzium ausscheidet, als er über den Darm resorbieren kann. Sollte dieser Zustand über längere Zeit bestehen bleiben, kommt es zum verstärkten Knochenabbau und einem sekundären Hyperparathyreodismus. Um das "fehlende" Kalzium aus dem Knochen zu moblisieren, wird in der Nebenschilddrüse "Parathormon" gebildet. Wie in Figur 2 dargestellt, stimuliert Parathormon den Knochenabbau und führt auf diese Weise zu einem Anstieg der Kalziumkonzentration im Blut. Die Kalziumkonzentration verbleibt im Blut somit relativ konstant zu Lasten des Knochens, der bei vorliegendem Kalziummangel für eine Erhöhung von Kalzium im Blut sein Kalzium spendet.

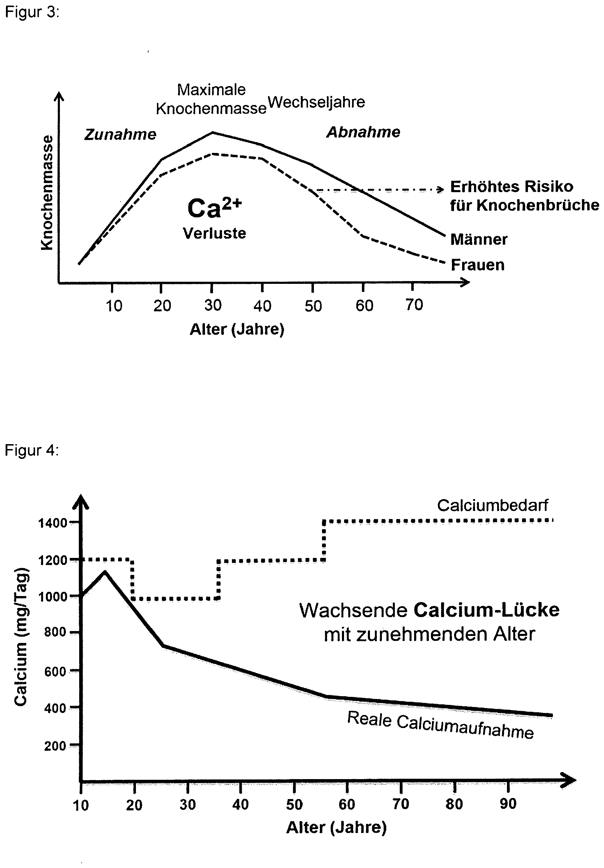

Figur 3 zeigt eine Darstellung über die "Kalziumverluste im Alter": Bereits nach dem 40. Lebensjahr beginnt ein schleichender Knochendichteverlust bei Männern und Frauen von 2-3% pro Jahr. Zu einer enormen Steigerung der jährlichen Verluste kann es bei mehr als 20% der Frauen nach der Menopause kommen. Oftmals unbemerkt verlieren die betroffenen Frauen mehr als ein Viertel ihrer Knochenmasse (=Kalzium) in einem relativ kurzen Zeitraum und haben dadurch ein sehr hohes Risiko für Frakturen im Laufe ihres weiteren Lebens. Die Erkrankung "Osteoporose" wird in vielen Fällen zu spät diagnostiziert - meistens erst dann, wenn bereits eine osteoporotische Fraktur (d.h. ohne traumatisches Ereignis) zum Beispiel an Wirbelkörper, Hüft- oder Handgelenk aufgetreten ist.

Ein nicht zu unterschätzendes und zusätzliches Problem der betroffenen Männer und Frauen im Alter ab 50 Jahren ist die wachsende Kalzium-Lücke, die sich aus einer progredienten negativen Kalziumbilanz ergibt. Wie in Figur 4 dargestellt ergibt sich im Alter eine zunehmende Diskrepanz zwischen dem täglichen Kalziumbedarf und der realen Aufnahme von Kalzium aus dem Gastrointestinaltrakt. Der altersbedingte hohe Bedarf an Kalzium wird im Wesentlichen durch folgende Faktoren beeinflusst: nachlassende Kalziumresorption im Gastrointestinaltrakt, kalziumarme Ernährung (wenig Milchprodukte), Vitamin D Mangel, wenig Bewegung, Kalziumspeicher "Knochen" soll wieder aufgefüllt werden (Aufbau neuer Knochenmasse), erhöhtes Parathormon im Blut (erhöhter Knochenabbau und Kalziumverluste) oder eingeschränkte Nierenfunktion (verminderte Kalzium-Rückresorption und weniger aktives Vitamin D).

Zum Ausgleich der natürlichen Kalziumverluste über Haut und Nieren sowie zur lebensnotwendigen Aufrechterhaltung eines konstanten Kalziumspiegels im Blut hat der menschliche Organismus letztendlich nur 2 Möglichkeiten:
1.) Kalzium aus dem "Kalziumspeicher Knochen" mobilisieren. Nachteil: Der dafür notwendige Abbau der Knochensubstanz kann langfristig zu Stabilitätsverlusten und Frakturen führen.
2.) Ausreichende Resorption von Kalzium aus dem Gastrointestinaltrakt und RückResorption von Kalzium in den Nieren. Beides wird durch Vitamin D gesteuert.

Die biologisch aktive Form des Vitamins D3 (1α,25-Dihydroxycholecalciferol; Calcitriol) fördert die Aufnahme von Kalzium aus dem Gastrointestinaltrakt ins Blut. Die maximale Serumkonzentration wird innerhalb von drei bis sechs Stunden nach der Aufnahme erreicht. In der Summe führt Vitamin D zu einer Förderung der Aufnahme von Kalzium in den Körper. Eine Überdosierung kann zu einer Vitamin D-Vergiftung führen, da der Körper Vitamin D speichert. Die Vitamin D-Vergiftung kann zu einer starken Demineralisierung des Knochens führen, welche zu Frakturen führt. Gleichzeitig können hohe Kalziumserumkonzentrationen zu einer anormalen Kalzifizierung der verschiedensten weichen Gewebe führen. Zusätzlich können aufgrund der erhöhten renalen Kalziumausscheidung Nierensteine entstehen.

Ein Vitamin D-Mangel führt aufgrund einer verminderten Resorption aus dem Darm dazu, dass weniger Kalzium aus der Nahrung ins Blut gelangt.

Ein Kalzium- und/oder Vitamin D-Mangel wird durch die Supplementation mit Kalzium- und Vitamin D-haltigen Präparaten behandelt. Die Anwendung von Kalzium und Vitamin D ist jedoch nicht unumstritten. Weltweit werden Diskussionen zur Verträglichkeit von Kalzium geführt und einige Wissenschaftler sehen in der Kalziumsupplementation ein kardiovaskuläres Risiko. Es wird behauptet, Kalzium erhöhe das Risiko für kardiovaskuläre Ereignisse wie Herzinfarkt. In Deutschland hat diese Diskussion dazu geführt, dass die Kalziumsupplementation häufig unterbleibt.

Für die Osteoporose bedeutet der Kalziummangel eine Verstärkung des Krankheitsbildes mit einer Erhöhung des Frakturrisikos.

Bei der manifesten Osteoporose ist der Knochenmineralgehalt vermindert (T-Score: <-2,5) und es haben sich Frakturen ereignet, beispielsweise 1 bis 3 Wirbelkörperfrakturen.

Eine Glukocorticoid-Langzeittherapie kann zu einer schweren glukocorticoid- oder corticoid-induzierten Osteoporose mit Frakturen führen. Bei einer Gabe von >5 mg Prednison-Äquivalent pro Tag über >3 Monate werden präventive bzw. therapeutische Maßnahmen empfohlen. Vor allem in den ersten sechs Monaten nach Therapiebeginn, bei erheblichen Dosissteigerungen im Verlauf und bei hochdosierter Langzeittherapie ist mit einer signifikanten Knochendichteabnahme zu rechnen, was in der Behandlung zu berücksichtigen ist. Der glukocorticoidinduzierte Knochenverlust hat mehrere Ursachen. Glukocorticoide führen am Beginn einer Therapie zu einer Steigerung der Knochenresorption. Steroide hemmen die Proliferation und Funktion von Osteoblasten und steigern deren Apoptose. Sie bewirken dadurch eine verminderte Knochenneubildung. Sie führen gleichzeitig zu einer negativen Kalziumbilanz durch Hemmung der intestinalen Kalziumresorption und Steigerung der Harn-Kalzium-Ausscheidung.

Solide primäre Tumore wie beispielsweise Mamma-, Prostata-, Lungen-, Darm- oder Knochenkarzinom (z.B. Osteosarkom) können sich im Knochen manifestieren und somit Knochenmetastasen bzw. Knochenerkrankungen hervorrufen. Knochenmetastasen sind Absiedlungen primärer Tumore in die Knochen und können dort zu Schmerzen und Brüchen führen. Verantwortlich ist ein verstärkter Knochenabbau oder eine überschießende Produktion von minderwertiger Knochensubstanz. Das verringert die Stabilität der Knochen. Tumorbedingte Knochenkomplikationen zeigen eine hohe Morbidität, eine erhöhte Knochenbruchrate, Nervenkompressionen und teilweise unerträgliche Schmerzen. Wird vermehrt Kalzium aus dem Knochen freigesetzt und ins Blut abgegeben, kann sich eine tumorinduzierte Hyperkalzämie entwickeln. So ist beispielsweise der Anteil an Patienten mit moderaten oder starken Knochenschmerzen (≥ 4 Punkte im BPI-SF [brief pain inventory, short form]) mit metastasiertem Mamma-, Prostata-, Lungen-, Darm- oder Knochenkarzinom (z.B. Osteosarkom) mit über 80%, gefolgt erst von neuralgischen Schmerzen mit ca. 10%, mit Abstand am höchsten (Cleeland, C.S. et al., Ann. Onc. 2005, 16: 972-980).

Der Begriff "Multiples Myelom" bzw. "Plasmozytom" oder "Kahler-Krankheit" beinhaltet eine Krebserkrankung des Knochenmarks, bei der die antikörperproduzierenden Zellen (Plasmazellen) stark vermehrt werden. Diese malignen Plasmazellen vermehren sich unkontrolliert und bilden funktionslose Antikörper oder Teile davon. Der Krankheitsverlauf kann sehr unterschiedlich sein mit moderaten bis zu hoch malignen Verläufen, die ohne Behandlung schnell zum Tod des Patienten führen. Die Symptome werden durch das Wachstum der Zellen verursacht oder durch die produzierten Antikörper bzw. deren Bruchstücken, die sich in Knochenschmerzen, Verringerung der Knochenmasse und in Frakturen manifestieren können, einhergehend mit einer erhöhten Freisetzung von Kalzium ins Blut, die zu tumor-induzierter Hyperkalzämie führen kann. Die Anzahl der Leukozyten nimmt ab, während sich die große Anzahl an Antikörpern im Gewebe ablagert und zu Funktionsstörungen vieler Organe, zu Nierenversagen und zur Beeinträchtigung der Durchblutung führt.

Arzneimittel zur Behandlung von Erkrankungen des Knochenstoffwechsels sind bekannt. Bei der medikamentösen Therapie der Osteoporose geht es in erster Linie darum den pathologischen Knochenmasseverlust zu stoppen. Die negative Bilanz (Ungleichgewicht zwischen An- und Abbau von Knochensubstanz) kann entweder antiresorptiv, durch eine Hemmung der Osteoklasten (Knochenabbau) oder anabol, durch eine Stimulation der Osteoblasten (Knochenaufbau) ausgeglichen werden. Erst wenn An- und Abbauprozesse wieder im Gleichgewicht zueinander stehen und eine ausreichende Versorgung mit Kalzium und Vitamin D gewährleistet wird, kann sich neue Knochenmasse aufbauen und das Risiko für osteoporotische Frakturen reduziert werden.

Zur Therapie der Osteoporose kann einerseits der osteoklastäre Knochenabbau durch Bisphosphonate wie Zoledronsäure gehemmt, andererseits durch anabole Medikamente der Knochenanbau stimuliert werden. Hier ist das rekombinante 1-34 Fragment des Parathormons zur Therapie der Osteoporose zugelassen.

Antiresorptiva wie Bisphosphonate sind derzeit Standard zur Behandlung metabolischer Knochenerkrankungen, die eine effektive Hemmung des Knochenabbaus erforderlich machen. Hierzu zählt der Einsatz dieser Substanzklassen zur Behandlung der Osteoporose wie auch -mit deutlich höheren Dosen und kürzeren Behandlungsintervallen- zur Prävention von tumorbedingten Skelettkomplikationen.

Bisphosphonate wie Zoledronsäure binden an den Knochen und werden von reifen Osteoklasten aufgenommen. Sie hemmen die Osteoklasten an der Knochenoberfläche, d.h. der Knochenabbau wird durch den Einsatz von Bisphosphonaten gehemmt.

Zoledronsäure bzw. deren Derivate werden üblicherweise verwendet, um die oben genannten Erkrankungen wie Osteoporose oder tumorbedingte Knochenerkrankungen zu behandeln.

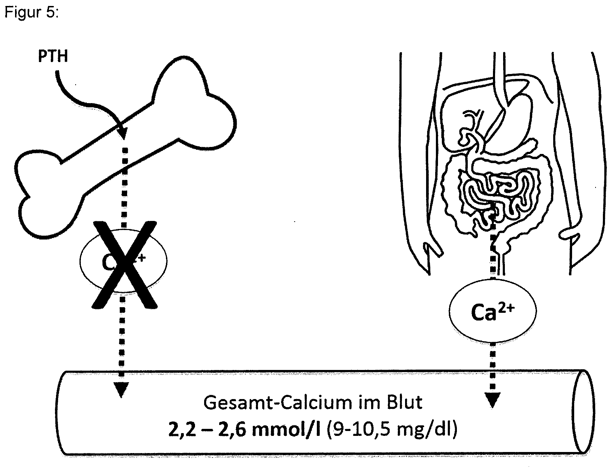

Als Folge der Anwendung von Zoledronsäure und Hemmung der Osteoklasten, verbleibt das Kalzium im Knochen. Eine Stimulation des Knochenabbaus z.B. durch Parathormon wird, in Abhängigkeit von der im Knochen vorliegenden Zoledronsäure-Moleküle, mehr oder weniger stark verhindert. Gerade bei hochdosierter Anwendung von Zoledronsäure (z.B. 4mg/4 Wochen) in der Onkologie besteht die Gefahr einer Tumortherapie-induzierten Hypokalzämie. Eine Hypokalzämie liegt vor, wenn das Gesamtkalzium im Blutserum unter 2,2 mmol/l (9 mg/dl) liegt. Durch die medikamentöse Hemmung der Osteoklasten kann die Kalziumkonzentration im Blut nur noch über eine Zufuhr von Kalzium von außen (oral oder intravenös) ausgeglichen werden. Dies ist in Figur 5 dargestellt. PTH aktiviert die Freisetzung von Kalzium aus dem Knochen, die allerdings durch die Hemmung der Osteoklasten durch die Zoledronsäure verhindert wird. Daher ist die Zufuhr von Kalzium von außen essentiell.

Die Hypokalzämie-Gefahr wird noch deutlich verstärkt, wenn ein sekundärer Hyperparathyreoidismus bei Vitamin D-Mangel vorliegt. Bei diesem häufigen Vitamin D-Mangelzustand wird zum Ausgleich des Serum-Kalziumspiegels vermehrt Parathormon ausgeschüttet. Parathormon erhöht indirekt die Kalziumkonzentration im Blutplasma durch Aktivierung der Osteoklasten.

Durch die antiresorptive Therapie wird die Hypokalzämie verstärkt, da die Osteoklasten gehemmt werden.

Insbesondere in der onkologischen Anwendung von Antiresorptiva wie Zoledronsäure kann sich eine Unterversorgung mit Kalzium und/oder Vitamin D auf dramatische Weise zuspitzen.

Auf diese Weise verursachte Hypokalzämien können zu Herzrhythmusstörungen und bei besonders schweren Verläufen sogar zum Tod führen, wenn die Hypokalzämie nicht rechtzeitig entdeckt und behandelt wird. Hypokalzämien bleiben zudem vielfach unentdeckt, da der Kalziumspiegel in der ambulanten klinischen Behandlung nicht in engen Zeitabständen als Laborparameter gemessen wird. Um die therapiebedingte Nebenwirkung der Zoledronsäure "Hypokalzämie" abzuwenden, wird vorliegend eine Supplementation mit Kalzium und Vitamin D bereitgestellt, die eine Hypokalzämie bei der Behandlung mit Zoledronsäure verhindern kann.

Eine unbeachtete Unterversorgung mit Kalzium und Vitamin D kann zudem den gesamten Erfolg einer antiresorptiven Therapie z.B. mit Zoledronsäure negativ beeinflussen und somit den gewünschten Schutz vor neuen Frakturen reduzieren bzw. die Anzahl der Frakturen sogar erhöhen, wenn die Kalzium und Vitamin D-Supplementation unter antiresorptiver Therapie unterlassen wird.

WO 2008/116133 A1 offenbart Dosierungsschemata, das Zoledronsäure und einige ihrer Derivate in Kombination mit Kalzium und Vitamin D beinhalten, um osteoporotische Frakturen zu reduzieren (Seite 2, [007]), sowie ein Multi-Komponenten-Kit für einen Patienten mit einem Risiko für sekundäre osteoporotische Frakturen und einem Vitamin D-Mangel, wobei das Kit Vitamin D oder deren funktionelle Analoge, Zoledronsäure oder ein funktionelles Analog und optional Kalzium-enthaltende Komponente enthält (Seite 2 [008]). WO 2008/116133 A1 offenbart nicht die Behandlung oder Prophylaxe von therapiebedingten Hypokalzämien, sondern schließt Patienten mit Hypokalzämien von der vorgeschlagenen Studie aus (Seiten 17-18, [0079]).

Die Patienteninformation zu Zometa (FDA-Prescribing Information, veröffentlicht unter: https://www.drugs.com/pro/zometa.html, Seite 5 unter dem Abschnitt "Hypocalcemia") offenbart u.a. die Hypokalzämie als mögliche Nebenwirkung bei der Behandlung mit Zometa, das den Wirkstoff Zoledronsäure enthält sowie den Hinweis, dass Kalzium gemessen werden und eine Hypokalzämie vor der Behandlung mit Zometa korrigiert werden sollte. Patienten sollten mit Kalzium und Vitamin D adäquat supplementiert werden. Einen Hinweis, wie die Patienten adäquat supplementiert werden sollen, offenbart die Patienteninformation nicht.

Die vorliegende Erfindung löst diese Probleme und stellt neue pharmazeutische Zusammensetzungen von Zoledronsäure und Kalzium und Vitamin D und/oder deren Derivaten bereit, um therapiebedingte Nebenwirkungen wie Hypokalzämien bei Erkrankungen des Knochenstoffwechsels besser zu behandeln bzw. diesen Erkrankungen vorzubeugen und auf diese Weise die optimale Wirkung sicherzustellen, sowie unsachgemäßen Gebrauch zu verhindern oder letzteren vorzubeugen.

### Zusammenfassung der Erfindung

In einem ersten Aspekt betrifft die Erfindung pharmazeutische Zusammensetzungen umfassend Zoledronsäure und Kalzium und Vitamin D zur Verwendung bei der Behandlung und/oder Prophylaxe der durch Zoledronsäure therapiebedingten Hypokalzämie bei der Behandlung von Osteoporose, postmenopausaler Osteoporose, manifester Osteoporose, Osteoporose des Mannes oder der Frau, dadurch gekennzeichnet, dass die pharmazeutische Zusammensetzung umfassend Zoledronsäure und/oder deren Derivat(e) in einer Dosierung von 4-6mg, stärker bevorzugt 5mg Zoledronsäure intravenös jährlich oder halbjährlich verabreicht wird und die pharmazeutische(n) Zusammensetzung(en) umfassend Kalzium mit einer Dosierung von 400-600mg pro Tag, besonders bevorzugt 500mg pro Tag oral verabreicht wird und Vitamin D in einer Dosierung von 800-1200 I.E. Vitamin D, besonders bevorzugt 1000 I.E. Vitamin D täglich oral verabreicht wird.

In einem weiteren Aspekt betrifft die Erfindung pharmazeutische Zusammensetzungen enthaltend Zoledronsäure und Kalzium und Vitamin D zur Verwendung bei der Behandlung und/ oder Prophylaxe der durch Zoledronsäure therapiebedingten Hypokalzämie bei skelettbezogenen Komplikationen, insbesondere pathologischen Frakturen, Bestrahlung des Knochens, Rückenmarkskompression oder operativen Eingriffen am Knochen, Knochenmetastasen, Schmerzen bei Knochenmetastasen, Nerveneinklemmungen oder Deformierungen aufgrund eines oder mehrerer solider Tumore wie beispielsweise Brustkrebs, Prostatakrebs, Lungenkrebs oder multiplem Myelom, dadurch gekennzeichnet, dass Zoledronsäure in einer Dosierung von 3-5mg, stärker bevorzugt 4mg Zoledronsäure intravenös zur 3-4 wöchigen Anwendung und 400-600mg Kalzium täglich, besonders bevorzugt 500mg Kalzium täglich und 800-1200 I.E. Vitamin D täglich, besonders bevorzugt 1000 I.E. Vitamin D täglich oral verabreicht werden.

Ferner betrifft die Erfindung in weiteren Aspekten die Verwendung von Derivaten der Zoledronsäure, die Behandlung und/oder Prophylaxe von therapiebedingten Nebenwirkungen, verschiedene Kalziumverbindungen und Vitamin D-Derivate zur Verwendung in den vorgenannten Ausführungsbeispielen, sowie pharmazeutisch geeignete Hilfsstoffe und Lösungsmittel.

### Ausführliche Beschreibung der Erfindung

Zoledronsäure ist ein sehr wirksames Antiresorptivum und wird zur Behandlung von Erkrankungen des Knochenstoffwechsels angewendet. Trotz ihrer Wirksamkeit ist die Behandlung mit Zoledronsäure durch erhebliche, teils schwere Nebenwirkungen belastet. Die vorliegende Erfindung stellt pharmazeutische Zusammensetzungen bereit, die die Behandlung mit Zoledronsäure in puncto Wirksamkeit und Sicherheit erheblich verbessern. Die pharmazeutischen Zusammensetzungen umfassen neben Zoledronsäure Kalzium und Vitamin D.

In der Fachinformation von Aclasta (Novartis Pharma, Arzneimittel, enthält 5mg Zoledronsäure als Infusionslösung, die einmal im Jahr verabreicht wird) wird lediglich eine ausreichende Einnahme von Kalzium und Vitamin D empfohlen, die genaue Dosierung wird nicht angegeben. Aclasta wird zur Behandlung der Osteoporose u.a. bei postmenopausalen Frauen und erwachsenen Männern mit einem erhöhten Risiko für Frakturen, einschließlich bei Patienten mit einer kürzlich erlittenen niedrig-traumatischen Hüftfraktur, zur Behandlung des Morbus Paget des Knochens bei Erwachsenen, sowie zur Behandlung der Osteoporose in Zusammenhang mit einer systemischen Langzeit Glukokortikoid-Therapie bei postmenopausalen Frauen und bei erwachsenen Männern mit einem erhöhten Frakturrisiko angewendet.

Zometa (Novartis Pharma, Arzneimittel, enthält 4mg/5ml oder 4mg/100ml Zoledronsäure als Infusionslösungskonzentrat oder Infusionslösung) wird zur Prävention skelettbezogener Komplikationen (pathologische Frakturen, Wirbelkörperkompressionen, Bestrahlung oder Operation am Knochen oder tumorinduzierte Hyperkalzämie) bei erwachsenen Patienten mit fortgeschrittenen, auf das Skelett ausgedehnte Tumorerkrankungen, sowie zur Behandlung von erwachsenen Patienten mit tumorinduzierter Hyperkalzämie mit 4mg alle 3-4 Wochen angewendet.

Die ausreichende tägliche Gesamtzufuhr an Kalzium beträgt 1000 mg. Über die Nahrung wird Kalzium aufgenommen, sodass bei kalziumreicher Ernährung die erforderliche Tagesdosis erreicht werden kann. Eine Kalziumzufuhr von mehr als 2500mg pro Tag wird als problematisch angesehen, weil sich dadurch unter anderem das Risiko für die Bildung von Nierensteinen erhöhen kann. Aufgrund diverser Veröffentlichungen über kardiovaskuläre Nebenwirkungen durch die übermäßige Einnahme von Kalzium, haben viele Patienten davon Abstand genommen, Kalzium in ausreichender Menge, selbst unter Behandlung mit Zoledronsäure, zu sich zu nehmen.

Vielfach wird dadurch der Behandlungserfolg von Zoledronsäure insgesamt gefährdet und die kardiovaskulären Nebenwirkungen nun durch die Unterversorgung mit Kalzium hervorgerufen bzw. durch die starke Hemmung der Osteoklasten durch die Zoledronsäurebehandlung, die eine Resorption des Kalziums aus dem Knochen ins Blut hemmt.

Dies führt zu Hypokalzämien, die kardiovaskuläre Nebenwirkungen hervorrufen. Im Prinzip wird damit genau das Gegenteil des erwünschten Erfolgs eintreten. Durch den Kalziummangel -nicht durch die Überversorgung mit Kalzium- treten nun vermehrt kardiovaskuläre Nebenwirkungen auf, die durch eine angemessene Versorgung mit Kalzium bei der Behandlung mit Zoledronsäure hätten verhindert werden können.

Eine Supplementation mit einer pharmazeutischen Zusammensetzung mit 1000-1500mg Kalzium pro Tag wird bei ausreichend kalziumhaltiger Ernährung zu einer Überversorgung mit Kalzium führen, die das Risiko der kardiovaskulären Nebenwirkungen erhöht. Entgegen der Studiendesigns, die in der Fachinformation von Aclasta eine Tagesdosis von 1000-1500mg Kalzium beinhalten, wird in der vorliegenden Erfindung eine niedrigere Dosierung bereitgestellt, um Hypokalzämien und kardiovaskuläre Nebenwirkungen zu behandeln bzw. diesen vorzubeugen.

Die bevorzugte Menge an Kalzium in der pharmazeutischen Zusammensetzung beträgt 400-600mg Kalzium pro Tag, besonders bevorzugt 500mg Kalzium pro Tag, um einerseits auch bei kalziumarmer Ernährung einen Grundspiegel an Kalzium zu erhalten und andererseits nicht in die Überversorgung zu geraten, die mit den genannten Risiken verbunden und demzufolge für die Patienten nicht akzeptabel ist. Dem Ergebnisbericht der in Deutschland durchgeführten "Nationale-Verzehrs-Studie II" zur Folge erreichen 46% der Männer und 55% der Frauen nicht die empfohlene tägliche Zufuhr von Kalzium. In der Altersgruppe zwischen 51-80 Jahre liegt die durchschnittliche Gesamtzufuhr in dem Bereich zwischen 490-1790 mg Kalzium pro Tag.

500mg Kalzium pro Tag sollen laut Fachinformation von Zometa eingenommen werden, allerdings in der Kombination mit 400 I.E. Vitamin D pro Tag. Die Studien für Zometa waren auf US-Patienten ausgerichtet. In den USA wird eine große Anzahl an Lebensmitteln mit Vitamin D supplementiert, so beispielsweise Milch, Orangensaft, Brotsorten oder Frühstücksflocken. In Deutschland und der EU ist dies im Wesentlichen nicht der Fall, so dass erfindungsgemäß eine höhere Menge an Vitamin D von 800-1200 I.E. pro Tag bereitgestellt wird, besonders bevorzugt 1000 I.E. Vitamin D pro Tag. Die höhere Menge an Vitamin D ist in der Lage, den Transport einer potentiell geringeren Menge an Kalzium aus dem Gastrointestinaltrakt ins Blut zu fördern und trotz der geringeren Menge an Kalzium zu einem ausreichenden Kalziumserumspiegel zu gelangen.

Der Vitamin D-Spiegel ist gut messbar. Die erfindungsgemäße Kombination wird für eine Vitamin D Serum-Konzentration bereitgestellt, die nicht unter 20ng/ml (50nmol/l Serum) liegt.

Bei einem schweren Mangel (<10ng/ml Serum) sollten 200.000 I.E. über 10 Tage und dann 20.000 I.E. wöchentlich substituiert werden. Bei einem deutlichen Mangel (10-20ng/ml Serum) sollte eine initiale Substitution von 100.000 I.E. erfolgen und dann eine Erhaltung von 20.000 I.E./Woche. Liegt ein Mangel (21-30ng/ml Serum) vor, sollte eine Substitution von 20.000 I.E./Woche erfolgen. Ist der normale Vitamin D-Spiegeln (31-60ng/ml Serum) erreicht, ist die tägliche erfindungsgemäße Substitution von 800-1200 I.E. Vitamin D, besonders bevorzugt 1000 I.E. anwendbar.

Eine Supplementation mit 400 I.E. Vitamin D ist nicht ausreichend, um den Vitamin D-Spiegel im normalen Bereich von 31-60ng/ml Serum bei Verabreichung der erfindungsgemäßen pharmazeutischen Zusammensetzung von Zoledronsäure und Kalzium zu halten.

Auf diese Weise werden durch die erfindungsgemäße pharmazeutische Zusammensetzung aus Zoledronsäure, Kalzium und Vitamin D Hypokalzämien verhindert.

Der Begriff "Zoledronsäure" oder "Zoledronat" (C₅H₁₀N₂O₇P₂; [1-Hydroxy-2-(imidazol-1-yl)ethyliden]diphosphonsäure, CAS-Nummer: 118072-93-8) umfasst zusätzlich alle bekannten pharmazeutisch verträglichen Derivate der Zoledronsäure. Dem Fachmann ist bekannt, dass Salze als Solvate wie beispielsweise Hydrate vorliegen können oder dass Zoledronsäure als Ester vorliegen kann. Zoledronsäure kann in jeder pharmazeutisch verträglichen Salz- oder Säureform bereitgestellt werden. Salze sind bevorzugt, da Membranen durch Salze weniger angegriffen werden. Bevorzugte Salze sind insbesondere, ohne darauf beschränkt zu sein, Zoledronsäure Dinatriumsalz Tetrahydrat (C₅H₈N₂Na₂O₇P₂•4H₂O, CAS-Nummer: 165800-07-7), Zoledronsäure Trinatriumsalz Hydrat (C₅H₇N₂Na₃O₇P₂•H₂O)₅, CAS-Nummer: 165800-08-8, Salze mit einwertigen Kationen, insbesondere Zoledronat-Natrium und/oder Ester, insbesondere Ethyl-, Propyl-, Isopropyl-, n-Butyl- und t-Butyl-Ester.

Erfindungsgemäß wird die pharmazeutische Zusammensetzung umfassend Zoledronsäure im Bereich Osteoporose, postmenopausaler Osteoporose, Osteoporose des Mannes oder der Frau mit 4-6mg, bevorzugt 5 mg pro Halb/-Jahr intravenös verabreicht.

Erfindungsgemäß wird die pharmazeutische Zusammensetzung umfassend Zoledronsäure im Bereich Onkologie mit 3-5mg, bevorzugt 4mg zur 3-4wöchigen Anwendung intravenös verabreicht.

Der Begriff "Kalzium" bzw. "Calcium" umfasst alle bekannten pharmazeutisch verträglichen Kalzium-Verbindungen, insbesondere ohne darauf beschränkt zu sein Kalziumzitrat, weitere Bezeichnungen: Tricalciumcitrat, TCC, Tricalciumdicitrat, Tricalcium-di-[2-hydroxy-1,2,3-propantricarboxylat]-Tetrahydrat, E 333, C₁₂H₁₀Ca₃O₁₄, CAS-Nummern: 813-94-5 (wasserfrei), 5785-44-4 (Tetrahydrat); Calciumgluconat-Monohydrat (C₁₂H₂₂CaO₁₄ • H₂O, CAS-Nummern: 299-28-5 (wasserfrei) und 66905-23-5 (Monohydrat)); Calciumlactatgluconat, ein Doppelsalz der Milch- und Gluconsäure, das als Gemisch vorliegt, weitere Bezeichnungen: CLG, Calciumlaktatglukonat, Calciumlaktoglukonat, E 327, E 578, CAS-Nummern: 11116-97-5, 814-80-2 (Calciumlactat-Pentahydrat), 18016-24-5 (Calciumgluconat-Monohydrat), oder Kalziumkarbonat CaCO₃, CAS-Nummer: 471-34-1, Kalzium-Phosphat, Kalzium-Dihydrogenphosphat, Kalzium-Hydrogenphosphat, Kalzium-Hydrogenphosphat Dihydrat, Kalzium-Acetat, Kalzium-Ascorbat, Kalzium-Chloride, Kalzium-Glucoheptonat, Kalzium-Glycerophosphat und/oder Kalzium-Sulfat.

Der Begriff "Vitamin D" umfasst Vitamin D oder dessen Derivate, insbesondere, ohne darauf beschränkt zu sein, Vitamin D3 (Cholecalciferol, C₂₇H₄₄O, CAS-Nummer: 67-97-0), Calcitriol (1,25-Dihydroxyvitamin D3, 1α,25-Dihydroxycholecalciferol, 1,25(OH)₂Vitamin D3, 1,25(OH)₂D3, (5Z,7E)-(1S,3R)-9,10-Seco cholesta-5,7,10(19)-trien-1,3,25-triol, CAS-Nummer: 32222-06-3) oder 1α,25-Dihydroxycholecalciferol (biologisch aktive Form von Vitamin D3), Alphacalcidol (1α-Hydroxyvitamin D3), 24,25-Dihydroxyvitamin D3 oder Calcifediol (25-Hydroxyvitamin D3 25-Hydroxycholecalciferol, CAS-Nummer: 19356-17-3, IUPAC-Name: (6R)-6-[(1R,3aR,4E,7aR)-4-[(2Z)-2-[(5S)-5-Hydroxy-2-methylidene-cyclohexylidene] ethylidene]-7a-methyl-2,3,3a,5,6,7-hexahydro-1H-inden-1-yl]-2-methylheptan-2-ol), Vitamin D2 (3β,5Z,7E,22E)-9,10-Secoergosta-5,7,10(19),22-tetraen-3-ol, Calciferol, Ergo-Calciferol, CAS-Nummer: 50-14-6 bzw. dessen biologisch aktive Formen.

Sowohl Vitamin D-Präparate, als auch Zoledronsäure und Kalzium sind käuflich erwerblich und die Verfahren zur Herstellung derselben der Fachperson bekannt.

Pharmazeutische Zusammensetzungen umfassend Zoledronsäure, Kalzium und Vitamin D zur Verwendung bei der Behandlung und/oder Prophylaxe der durch Zoledronsäure therapiebedingten Hypokalzämie bei der Behandlung von Osteoporose, postmenopausaler Osteoporose, manifester Osteoporose, Osteoporose des Mannes oder der Frau, die in einer Zoledronsäure-Konzentration von 4-6mg jährlich oder halbjährlich, stärker bevorzugt 5mg jährlich oder halbjährlich und 400-600mg Kalzium täglich, besonders bevorzugt 500mg Kalzium täglich und 800-1200 I.E. Vitamin D, besonders bevorzugt 1000 I.E. Vitamin D täglich oral verabreicht bereitgestellt werden.

In einem weiteren Ausführungsbeispiel werden pharmazeutische Zusammensetzungen umfassend 3-5mg, stärker bevorzugt 4mg Zoledronsäure intravenös zur 3-4 wöchigen Anwendung und 400-600mg Kalzium täglich, besonders bevorzugt 500mg Kalzium täglich und 800-1200 I.E. Vitamin D, besonders bevorzugt 1000 I.E. Vitamin D zur Verwendung bei der Behandlung und/oder Prophylaxe der durch Zoledronsäure therapiebedingten Hypokalzämie bei skelettbezogenen Komplikationen, insbesondere pathologischen Frakturen, Rückenmarkskompressionen, Bestrahlung oder Operation am Knochen, Schmerzen bei Knochenmetastasen aufgrund von soliden Tumoren wie beispielsweise Mammakarzinom, Prostatakarzinom, Lungenkrebs, Multiplem Myelom bereitgestellt.

In einem Ausführungsbeispiel werden die oben angegebenen Mengen Zoledronsäure in einer Infusionslösung von 5ml bis 100ml vor, stärker bevorzugt als 5ml Infusionslösungskonzentrat oder als 100ml Fertiginfusionslösung in einer pharmazeutisch geeigneten Lösung wie beispielsweise isotonischer Kochsalzlösung, isotonischer Mannitol-Natriumcitrat-Lösung oder anderen pharmazeutisch geeigneten isotonischen Lösungen bereitgestellt.

In einem weiteren Ausführungsbeispiel werden Kalzium und Vitamin D mit pharmazeutisch geeigneten Hilfsstoffen bereitgestellt, insbesondere Lactose, Stärke, Säuerungsmitteln insbesondere Zitronensäure und Apfelsäure, Säureregulatoren insbesondere Natriumhydrogencarbonat und Natriumcarbonat, Feuchthaltemitteln insbesondere Sorbitol, Xylitol und Inulin, Trennmitteln insbesondere Tricalciumphosphat, Speisefettsäuren insbesondere Magnesiumsalze insbesondere Magnesiumstearat, natürliche und naturidentische und andere Aromen und Aromastoffe, Süßstoffe insbesondere Natrium-Cyclamat, Aspartam und Saccharin-Natrium, Maltodextrin, Farbstoffe insbesondere Rote-Bete-Saftpulver und Riboflavin-5'-phosphat, Siliciumdioxid insbesondere hochdispers, Siliciumdioxid hydrat, Phenylalanin, Arabisches Gummi, Saccharose, Gelatine, Maisstärke, Sojaöl, Glycerol, DL-alpha-Tocopherol, Isomalt, Natriumhydrogencarbonat, Natriumdihydrogencarbonat, Natriumcitrat, Natriumdihydrogencitrat, Carmellose Natrium, Acesulfam Kalium, Natriumascorbat, Triglyceride insbesondere mittelkettige, und/oder in pharmazeutisch verträglichen Flüssigkeiten, insbesondere Wasser, isotonischer Kochsalz- oder Glucoselösung oder anderen pharmazeutisch geeigneten Lösungen bereitgestellt.

In einem weiteren Ausführungsbeispiel werden Zoledronsäure, Kalzium und/oder Vitamin D, gegebenenfalls mit pharmazeutisch geeigneten Hilfsstoffen und/oder Flüssigkeiten, insbesondere als Brausetabletten, Schlucktabletten oder -kapseln, Kautabletten, Brausegranulaten, Direktgranulaten, Trinklösungen, Tropfen, Sublingualsprays, als Infusionslösungskonzentrate, Fertiginfusionen, Injektionslösungskonzentraten, Injektionslösungen oder Fertigspritzen einzeln oder zusammen bereitgestellt.

Die nachfolgenden Figuren und Beispiele dienen der Erläuterung ohne jedoch die Erfindung auf die Figuren oder Beispiele einzuschränken.

### Figuren:

**Figur 1****:** zeigt eine Darstellung über die Kalziumzufuhr vom Gastrointestinaltrakt ins Blut und in den Knochen, sowie die beeinflussenden Faktoren, die eine Kalziumaufnahme fördern bzw. verringern.
**Figur 2****:** zeigt die Folgen einer insuffizienten Kalziumaufnahme durch die Nahrung.
**Figur 3****:** zeigt die Zu- und Abnahme der Knochenmasse im Verlauf des menschlichen Lebens und das erhöhte Knochenbruchrisiko im Alter mit Abnahme der Knochenmasse.
**Figur 4****:** zeigt den erhöhten Kalziumbedarf mit zunehmenden Alter durch verringerte reale Kalziumaufnahme.
**Figur 5****:** Eine Hypokalzämie liegt vor, wenn das Gesamtkalzium im Blutserum unter 2,2 mmol/l (9 mg/dl) liegt. Durch die medikamentöse Hemmung der Osteoklasten kann die Kalziumkonzentration im Blut nur noch über eine Zufuhr von Kalzium von außen (oral oder intravenös) ausgeglichen werden. Dies ist in Figur 5 dargestellt.

### Beispiel 1:

Ein Vitamin D-Mangel ist eine häufige Diagnose in der osteologischen Praxis. Zu Beginn der Therapie wiesen 89 von 423 Patienten einen Vitamin D-Mangel auf, der sich unter dem Wert von 20ng/ml Serum bzw. 50 nmol/l befand. Die Erhaltungsdosis betrug 1000 I.E. täglich Vitamin D. Eine Erhaltungsdosis von 400 I.E. Vitamin D täglich war nicht ausreichend, den Vitamin D-Spiegel auf dem Normwert zu halten.

Tabelle 1 zeigt, dass eine Erhaltungsdosis von 1000 I.E. Vitamin D geeignet ist, den Vitamin D-Spiegel im Normbereich zu halten:

**Tabelle 1:**

| Patient | Vitamin D-Anfangswert | Vitamin D-Wert bei Erhaltungsdosis 1000 I.E. Vitamin D täglich |
|---|---|---|
| 85 Jahre, weiblich | 9,4ng/ml | 25,5 ng/ml |
| 65 Jahre, weilblich | 8,8ng/ml | 23,4 ng/ml |
| 77 Jahre, weiblich | 12,4 ng/ml | 29,2 ng/ml |
| 81 Jahre, weiblich | 10,4ng/ml | 30,4 ng/ml |
| 75 Jahre, weiblich | 14,6ng/ml | 28,8 ng/ml |
| 86 Jahre, weiblich | 10,9ng/ml | 23,7ng/ml |
| 81 Jahre, weiblich | 8,6 ng/ml | 23,2ng/ml |
| 71 Jahre, weiblich | 17,1 ng/ml | 30,5 ng/ml |

### Beispiel 2:

Der Vitamin D-Spiegel von 37 weiteren Patienten wurde analysiert. Zu Beginn der Therapie lag bei etwa 51% der Patienten (n=19) ein Vitamin D Mangel [25(OH)VitD - Serumspiegel < 30ng/ml Serum bzw. 75 nmol/l] vor. Bei 27% der Patienten (n=10) lag der 25(OH)VitD - Serumspiegel unter 20ng/ml Serum bzw. 50 nmol/l (=schwerer Vitamin D Mangel). Die folgende Tabelle 2 zeigt in der jeweils linken Spalte die tägliche Aufnahme an Kalzium durch die Nahrung im mg. Die tägliche Zufuhr von weniger als 1000mg durch Nahrungsmittel führt zu einer negativen Kalziumbilanz. Daraus entwickelt sich ein sekundärer Hyperparathyreodismus und Knochensubstanz geht verloren. Eine tägliche Versorgung von weniger als 500mg Kalzium ist mit einem erhöhten Frakturrisiko für nicht-vertebrale Frakturen verbunden.

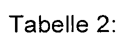

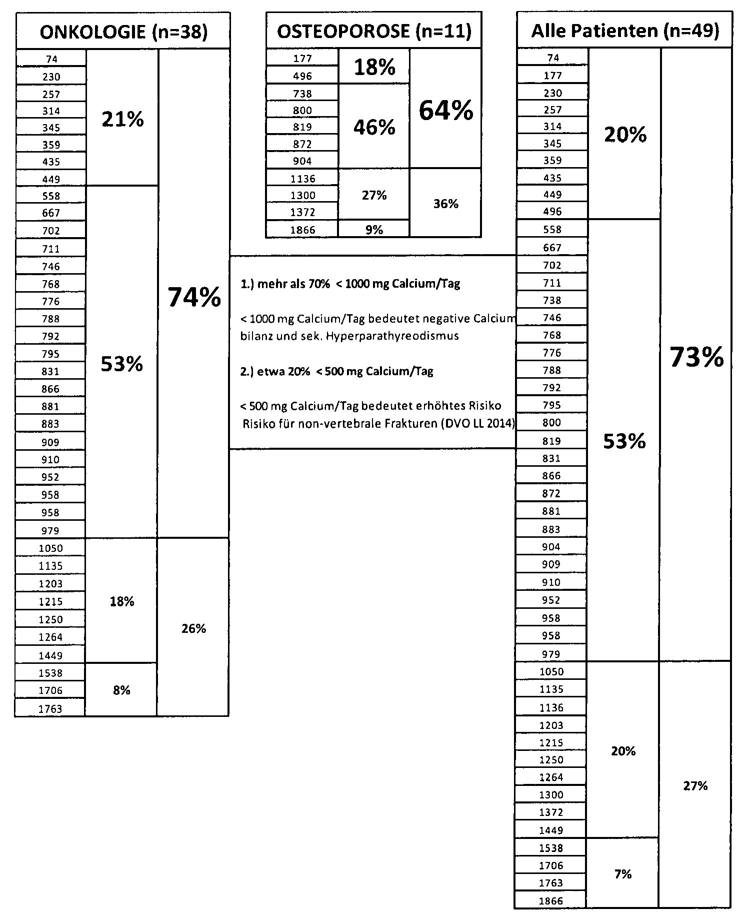

### Beispiel 3:

Die folgende Patientenkasuistik zeigt, dass die erfindungsgemäße pharmazeutische Zusammensetzung von 5mg Zoledronsäure jährlich intravenös verabreicht, 500mg Kalzium täglich und 1000 I.E. Vitamin D täglich oral eingenommen, eine therapiebedingte Hypokalzämie, verursacht durch die Behandlung mit Zoledronsäure, wirksam verhindert. Die Werte stammen von einer 73 Jahre alten Patientin mit manifester Osteoporose und einer vorausgegangenen niedrig-traumatischen Wirbelkörperfraktur. Sowohl die Kalzium-Werte als auch die Werte für Vitamin D liegen bei der Behandlung mit den erfindungsgemäßen pharmazeutischen Zusammensetzungen im Normbereich.

### Folgende Tabelle 3: Patientenkasuistik

| Datum | Ca in mmol/l | 25(OH) Vitamin D3-Serumspiegel in ng/ml |
|---|---|---|
| 21.08.12 | 2,21 | 30,40 |
| 12.03.13 | 2,36 | 28,40 |
| 08.08.13 | 2,27 | 47,30 |
| 18.02.14 | 2,27 | 34,30 |
| 24.07.14 | 2,23 | 34,30 |
| 19.03.15 | 2,25 | 39,10 |
| 23.02.16 | 2,32 | 29,00 |

### Beispiel 4:

Die folgenden Daten von 10 onkologischen Patienten (Prostatakarzinom oder Mamakarzinom) in Tabelle 5 zeigen die Serumspiegel von Kalzium und Vitamin D3 bei Verabreichung von monatlich 4 mg Zoledronsäure, sowie der oralen Einnahme von 500 mg Kalzium täglich und 1000 I.E. Vitamin D3 täglich.

**Tabelle 4: Die Patienten erhielten 4mg Zoledronsäure/Monat, 500mg Kalzium-Supplementation täglich und 1000 I.E. Vitamin D3 täglich:**

| Patient/- in | Kalzium mmol/l | 25(OH) Vitamin D3-Serumspiegel µg/l | nach Wochen | Kalzium | 25(OH) Vitamin D3-Serumspiegel; µg/l | Kalzium in Ernährung mg/Tag |
|---|---|---|---|---|---|---|
| 1 | 2,09 | 17,2 | 16 | 2,14 | 37,1 | 1449 |
| 2 | 2,4 | 17,2 | 4 | 2,31 | 24,3 | 979 |
| 3 | 2,02 | 12,1 | 16 | 2,22 | 25,2 | 958 |
| 4 | 2,37 | Keine Angabe | 8/16/20 | 2,46/2,46/2,32 | 33,1 nach 20 Wochen | 702 |
| 5 | 2,36 | Keine Angabe | 4/16/24 | 2,41/2,4/2,54 | 22,3 nach 20 Wochen | 230 |
| 6 | 2,4 | Keine Angabe | 4/8/16 | 2,26/2,41/2,4 | Keine Angabe/19,8/22,8 | 952 |
| 7 | 2,47 | Keine Angabe | 4/9/13 | 2,26/2,4/2,36 | 40,3 nach 13 Wochen | 1215 |
| 8 | 2,42 | 22 | 8/12/16 | 2,34/2,41/2,4 | Keine Angabe | 909 |
| 9 | 2,48 | 46,7 | 4/8/12/16 | 2,3/ 2,34/ 2,38/2,41 | 46,2/ keine Angabe/ keine Angabe/ 48,1 | 544 |
| 10 | 2,25 | Keine Angabe | 4/8/12/16 | 2,31/ 2,29/ 2,34/2,28 | Keine Angabe | 1250 |

**Tabelle 5: Die folgenden Patienten in Tabelle 5 waren an Osteoporose erkrankt und erhielten 5 mg Zoledronsäure/Jahr, 500mg Kalzium-Supplementation täglich und 1000 I.E. Vitamin D3 täglich:**

| Patient/- in | Kalzium mmol/l | 25(OH) Vitamin D3-Serumspiegel; µg/l | nach Wochen | Kalzium | 25(OH) Vitamin D3-Serumspiegel; µg/l | Kalzium in Ernährung mg/Tag |
|---|---|---|---|---|---|---|
| 11 | 2,28 | 23,1 | 24 | 2,24 | 40,1 | 872 |
| 12 | 2,41 | 15 | 24 | 2,4 | 25,8 | 1866 |
| 13 | 2,4 | 21,5 | 24 | 2,37 | 28,6 | 738 |
| 14 | 2,32 | 31,3 | 24 | 2,44 | 45,4 | 800 |

Bei einem 25(OH) Vitamin D - Serumspiegel < 20ng/ml liegt ein schwerer Vitamin D-Mangel vor, der nach der DVO-Leitlinie ein erhöhtes Frakturrisiko bedeutet. Die Daten in Beispiel 5 zeigen, dass die erfindungsgemäße Kombination geeignet ist, den Vitamin D- Spiegel über 20ng/ml bzw. 20µg/l zu normalisieren und dadurch das Frakturrisiko zu senken.

Der Kalzium-Serumspiegel ist bei Patienten mit Osteoporose in der Regel im Normbereich. Bei onkologischen Patienten kann die Kalziumkonzentration im Blut ansteigen (=tumorinduzierte Hyperkalzämie) oder therapiebedingt absinken (therapie-induzierte Hypokalzämie). Patienten mit einer kalziumarmen Ernährung haben deshalb ein zusätzliches Hypokalzämie-Risiko. Eine erfindungsgemäße tägliche Supplementation mit 500 mg Kalzium sogt für eine ausgeglichene Kalziumbilanz.

Ein erhöhter PTH-Spiegel (=sekundärer Hyperparathyreodisumus) sowie eine Hypokalzämie - induziert durch eine anti-resorptive Therapie mit Zoledronsäure - sind Risikofaktoren für die Entstehung einer Nekrose am Kieferknochen.

Die Patienten, die mit der erfindungsgemäßen Kombination behandelt wurden, waren frei von Nebenwirkungen, insbesondere traten keine Kieferosteonekrosen, nicht heilende Wunden bis zur Auflösung des Kiefers, Hypokalzämien, kardiovaskuläre Nebenwirkungen wie Herzinfarkt, Vorhofflimmern, Herzrhythmusstörungen, Krämpfe und/oder Taubheitsgefühl auf.

Es traten ferner keine skelettbezogenen Komplikationen, insbesondere keine pathologischen Frakturen, Bestrahlungen des Knochens, Rückenmarkskompressionen oder operative Eingriffe am Knochen, Knochenmetastasen, Schmerzen bei Knochenmetastasen, Nerveneinklemmungen oder Deformierungen aufgrund eines oder mehrerer solider Tumore wie beispielsweise Brustkrebs, Prostatakrebs, Lungenkrebs oder multiplem Myelom auf, sowie keine Frakturen.

## Patentansprüche

1. Pharmazeutische Zusammensetzungen umfassend Zoledronsäure und Kalzium und Vitamin D zur Verwendung bei der Behandlung und/oder Prophylaxe der durch Zoledronsäure therapiebedingten Hypokalzämie bei der Behandlung von Osteoporose, postmenopausaler Osteoporose, manifester Osteoporose, Osteoporose des Mannes oder der Frau, **dadurch gekennzeichnet, dass** die pharmazeutische Zusammensetzung umfassend Zoledronsäure in einer Dosierung von 4-6mg, stärker bevorzugt 5mg Zoledronsäure intravenös jährlich oder halbjährlich verabreicht wird und die pharmazeutische(n) Zusammensetzung(en) umfassend Kalzium mit einer Dosierung von 400-600mg pro Tag, besonders bevorzugt 500mg pro Tag oral verabreicht wird und Vitamin D in einer Dosierung von 800-1200 I.E. Vitamin D, besonders bevorzugt 1000 I.E. Vitamin D täglich oral verabreicht wird.

2. Pharmazeutische Zusammensetzungen enthaltend Zoledronsäure und Kalzium und Vitamin D zur Verwendung bei der Behandlung und/oder Prophylaxe der durch Zoledronsäure therapiebedingten Hypokalzämie bei skelettbezogenen Komplikationen, insbesondere pathologischen Frakturen, Bestrahlung des Knochens, Rückenmarkskompression oder operativen Eingriffen am Knochen, Knochenmetastasen, Schmerzen bei Knochenmetastasen, Nerveneinklemmungen oder Deformierungen aufgrund eines oder mehrerer solider Tumore wie beispielsweise Brustkrebs, Prostatakrebs, Lungenkrebs oder multiplem Myelom, **dadurch gekennzeichnet, dass** Zoledronsäure in einer Dosierung von 3-5mg, stärker bevorzugt 4mg Zoledronsäure intravenös zur 3-4 wöchigen Anwendung und 400-600mg Kalzium täglich, besonders bevorzugt 500mg Kalzium täglich und 800-1200 I.E. Vitamin D täglich, besonders bevorzugt 1000 I.E. Vitamin D täglich oral verabreicht werden.

3. Pharmazeutische Zusammensetzungen enthaltend Zoledronsäure und Kalzium und Vitamin D zur Verwendung nach Anspruch 1-2, wobei Zoledronsäure pharmazeutisch geeignete Salze und/oder Ester umfasst, insbesondere Salze mit einwertigen Kationen, insbesondere Zoledronat-Natrium und/oder Ester, insbesondere Ethyl-, Propyl-, Isopropyl, n-Butyl- und t-Butyl-Ester.

4. Pharmazeutische Zusammensetzungen enthaltend Zoledronsäure und Kalzium und Vitamin D zur Verwendung nach Anspruch 1-3, wobei Kalzium insbesondere Kalziumlaktatgluconat, Kalziumgluconat-Monohydrat, Kalziumlaktat-Pentahydrat, Kalziumzitrat, Kalziumzitrat-Tetrahydrat, Kalziumkarbonat, Kalzium-Phosphat, Kalzium-Dihydrogenphosphat, Kalzium-Hydrogenphosphat, Kalzium-Hydrogenphosphat Dihydrat, Kalzium-Acetat, Kalzium-Ascorbat, Kalzium-Chloride, Kalzium-Glucoheptonat, Kalzium-Glycerophosphat und/oder Kalzium-Sulfat umfasst.

5. Pharmazeutische Zusammensetzungen enthaltend Zoledronsäure und Kalzium und Vitamin D zur Verwendung nach Anspruch 1-4, wobei Vitamin D insbesondere Vitamin D und/oder Vitamin D3 und/oder Vitamin D2 und/oder deren Derivate, insbesondere Calcitriol (1,25-Dihydroxyvitamin D3) oder 1α, 25-Dihydroxycholecalciferol, Alphacalcidol (1α-Hydroxyvitamin D3), 24,25-Dihydroxyvitamin D3, Calcifediol, Vitamin D2 und/oder Ergocalciferol umfasst.

6. Pharmazeutische Zusammensetzungen enthaltend Zoledronsäure und Kalzium und Vitamin D zur Verwendung nach den Ansprüchen 1-5, zusätzlich umfassend pharmazeutisch geeignete Hilfsstoffe und/oder Flüssigkeiten oder Lösungsmittel.

7. Pharmazeutische Zusammensetzungen enthaltend Zoledronsäure und Kalzium und Vitamin D zur Verwendung nach den Ansprüchen 1-6, wobei die Hilfsstoffe für die pharmazeutischen Zusammensetzungen Lactose, Stärke, Säuerungsmittel insbesondere Citronensäure, Apfelsäure, Säureregulatoren insbesondere Natriumhydrogencarbonat und Natriumcarbonat, Feuchthaltemitteln insbesondere Sorbitol, Xylitol und Inulin, Trennmitteln insbesondere Tricalciumphosphat, Speisefettsäuren insbesondere Magnesiumsalze insbesondere Magnesiumstearat, natürliche und naturidentische und andere Aromen und Aromastoffe, Süßstoffe insbesondere Natrium-Cyclamat, Aspartam und Saccharin-Natrium, Maltodextrin, Farbstoffe insbesondere Rote-Bete-Saftpulver und Riboflavin-5'-phosphat, Siliciumdioxid insbesondere hochdispers, Siliciumdioxid hydrat, Phenylalanin, Arabisches Gummi, Saccharose, Gelatine, Maisstärke, Sojaöl, Glycerol, DL-alpha-Tocopherol, Isomalt, Natriumhydrogencarbonat, Natriumdihydrogencarbonat, Natriumcitrat, Natriumdihydrogencitrat, Carmellose Natrium, Acesulfam Kalium, Natriumascorbat, Triglyceride insbesondere mittelkettige, und/oder in pharmazeutisch verträglichen Flüssigkeiten, insbesondere Wasser, isotonischer Kochsalz- oder Glucoselösung umfassen.

8. Pharmazeutische Zusammensetzungen enthaltend Zoledronsäure und Kalzium und Vitamin D zur Verwendung den Ansprüchen 1-7, wobei die pharmazeutisch geeigneten Lösungsmittel oder Flüssigkeiten Wasser, isotonische Kochsalzlösung, isotonische Mannitol-Natriumacetatlösung oder Glucoselösung umfassen.

9. Pharmazeutische Zusammensetzungen enthaltend Zoledronsäure und Kalzium und Vitamin D zur Verwendung nach den Ansprüchen 1-8, wobei die pharmazeutische Zusammensetzung umfassend Zoledronsäure als Infusion, Infusionslösungskonzentrat, Fertiginfusion oder Fertigspritze und die pharmazeutischen Zusammensetzungen umfassend Kalzium und/oder Vitamin D in fester oder flüssiger Form als Brausetablette, Schlucktablette oder -kapsel, Kautablette, Brausegranulat, Direktgranulat, Trinklösung, Tropfen, Sublingualspray, einzeln oder zusammen bereitgestellt wird.

## Claims

1. Pharmaceutical compositions comprising zoledronic acid and calcium and vitamin D for use in the treatment and/or prophylaxis of treatment-related hypocalcaemia due to zoledronic acid during treatment of osteoporosis, postmenopausal osteoporosis, manifest osteoporosis, osteoporosis in men or women, **characterized in that** the pharmaceutical composition comprising zoledronic acid is administered intravenously at a dosage of 4-6 mg, more preferably 5 mg, zoledronic acid once- or twice-yearly and that the pharmaceutical composition(s) comprising calcium is/are administered orally at a dosage of 400-600 mg per day, more preferably 500 mg per day, and that vitamin D is administered orally at a daily dosage of 800-1200 IU vitamin D, more preferably 1000 IU vitamin D.

2. Pharmaceutical compositions comprising zoledronic acid and calcium and vitamin D for use in the treatment and/or prophylaxis of treatment-related hypocalcaemia due to zoledronic acid in skeletal-related complications, in particular pathological fractures, irradiation of the bone, spinal cord compression or surgical interventions in bone, bone metastases, pain in bone metastases, nerve entrapment or deformations as a result of one or more solid tumours, for example breast cancer, prostate cancer, lung cancer or multiple myeloma, **characterized in that** zoledronic acid at a dosage of 3-5 mg, more preferably 4 mg of zoledronic acid intravenously for a 3-4 week period of use and 400-600 mg calcium daily, more preferably 500 mg calcium daily, and 800-1200 IU vitamin D daily, more preferably 1000 IU vitamin D daily, are administered orally.

3. Pharmaceutical compositions comprising zoledronic acid and calcium and vitamin D for use according to Claim 1-2, wherein zoledronic acid includes pharmaceutically suitable salts and/or esters, in particular salts having monovalent cations, in particular zoledronate sodium and/or esters, in particular ethyl, propyl, isopropyl, n-butyl and t-butyl esters.

4. Pharmaceutical compositions comprising zoledronic acid and calcium and vitamin D for use according to Claim 1-3, wherein calcium includes in particular calcium lactate gluconate, calcium gluconate monohydrate, calcium lactate pentahydrate, calcium citrate, calcium citrate tetrahydrate, calcium carbonate, calcium phosphate, calcium dihydrogen phosphate, calcium hydrogen phosphate, calcium hydrogen phosphate dihydrate, calcium acetate, calcium ascorbate, calcium chloride, calcium glucoheptonate, calcium glycerophosphate and/or calcium sulfate.

5. Pharmaceutical compositions comprising zoledronic acid and calcium and vitamin D for use according to Claim 1-4, wherein vitamin D includes in particular vitamin D and/or vitamin D3 and/or vitamin D2 and/or derivatives thereof, in particular calcitriol (1,25-dihydroxyvitamin D3) or 1α,25-dihydroxycholecalciferol, alfacalcidol (1α hydroxyvitamin D3), 24,25-dihydroxyvitamin D3, calcifediol, vitamin D2 and/or ergocalciferol.

6. Pharmaceutical compositions comprising zoledronic acid and calcium and vitamin D for use according to Claims 1-5, additionally including pharmaceutically suitable excipients and/or fluids or solvents.

7. Pharmaceutical compositions comprising zoledronic acid and calcium and vitamin D for use according to Claims 1-6, wherein the excipients for the pharmaceutical compositions include lactose, starch, acidification agents, in particular citric acid, malic acid, acidity regulators, in particular sodium hydrogen carbonate and sodium carbonate, humectants, in particular sorbitol, xylitol and inulin, release agents, in particular tricalcium phosphate, fatty acids, in particular magnesium salts, in particular magnesium stearate, natural and nature-identical and other flavours and flavourings, sweeteners, in particular sodium cyclamate, aspartame and saccharin sodium, maltodextrin, colorants, in particular beetroot juice powder and riboflavin-5' phosphate, silica, in particular colloidal hydrated silica, phenylalanine, gum arabic, sucrose, gelatin, maize starch, soybean oil, glycerol, DL-alpha-tocopherol, isomalt, sodium hydrogen carbonate, sodium dihydrogen carbonate, sodium citrate, sodium dihydrogen citrate, carmellose sodium, acesulfame potassium, sodium ascorbate, triglycerides, in particular medium-chain, and/or in pharmaceutically compatible fluids, in particular water, isotonic saline or isotonic glucose solution.

8. Pharmaceutical compositions comprising zoledronic acid and calcium and vitamin D for use according to Claims 1-7, wherein the pharmaceutically compatible solvents or fluids include water, isotonic saline, isotonic mannitol-sodium acetate solution or glucose solution.

9. Pharmaceutical compositions comprising zoledronic acid and calcium and vitamin D for use according to Claims 1-8, wherein, individually or together, the pharmaceutical composition comprising zoledronic acid is provided as an infusion, concentrate for solution for infusion, ready-to-use solution for infusion or prefilled syringe, and the pharmaceutical compositions comprising calcium and/or vitamin D in solid or liquid form are provided as an effervescent tablet, tablet or capsule to be swallowed, chewable tablet, effervescent granules, oral granules, oral solution, drops, or sublingual spray.

## Revendications

1. Compositions pharmaceutiques comprenant de l'acide zolédronique et du calcium et de la vitamine D, destinées à l'utilisation dans le traitement et/ou la prophylaxie de l'hypocalcémie due à un traitement par l'acide zolédronique dans le traitement de l'ostéoporose, l'ostéoporose postménopausique, l'ostéoporose manifeste, l'ostéoporose de l'homme ou de la femme, **caractérisées en ce que** la composition pharmaceutique comprenant de l'acide zolédronique est administrée par voie intraveineuse une ou deux fois par an à une posologie de 4-6 mg, de façon plus particulièrement préférée 5 mg d'acide zolédronique et la/les composition(s) pharmaceutique(s) comprenant du calcium est/sont administrée(s) par voie orale à une posologie de 400-600 mg par jour, de façon particulièrement préférée 500 mg par jour et de la vitamine D est administrée par voie orale à une posologie de 800-1200 U.I. de vitamine D, de façon particulièrement préférée 1000 U.I. par jour.

2. Compositions pharmaceutiques contenant de l'acide zolédronique et du calcium et de la vitamine D, destinées à l'utilisation dans le traitement et/ou la prophylaxie de l'hypocalcémie due à un traitement par l'acide zolédronique dans le cas de complications osseuses, en particulier de fractures pathologiques, d'irradiation de l'os, de compression médullaire ou d'interventions chirurgicales sur l'os, de métastases osseuses, de douleurs associées à des métastases osseuses, de compressions de nerfs ou de déformations dues à une ou plusieurs tumeurs solides, comme par exemple cancer du sein, cancer de la prostate, cancer du poumon ou myélome multiple, **caractérisées en ce que** sont administrés de l'acide zolédronique en une posologie de 3-5 mg, de façon particulièrement préférée 4 mg d'acide zolédronique par voie intraveineuse en utilisation toutes les 3-4 semaines et 400-600 mg de calcium une fois par jour, de façon particulièrement préférée 500 mg de calcium une fois par jour et 800-1200 U.I. de vitamine D une fois par jour, de façon particulièrement préférée 1000 U.I. de vitamine D une fois par jour, par voie orale.

3. Compositions pharmaceutiques contenant de l'acide zolédronique et du calcium et de la vitamine D, destinées à l'utilisation selon l'une quelconque des revendications 1 et 2, dans lesquelles l'acide zolédronique comprend les sels et/ou esters pharmaceutiquement acceptables, en particulier les sels avec des cations monovalents, en particulier le zolédronate de sodium et/ou des esters, en particulier les esters éthylique, propylique, isopropylique, n butylique et tert-butylique.

4. Compositions pharmaceutiques contenant de l'acide zolédronique et du calcium et de la vitamine D, destinées à l'utilisation selon l'une quelconque des revendications 1-3, dans lesquelles le calcium comprend en particulier le lactate-gluconate de calcium, le gluconate de calcium monohydraté, le lactate de calcium pentahydraté, le citrate de calcium, le citrate de calcium tétrahydraté, le carbonate de calcium, le phosphate de calcium, le dihydrogénophosphate de calcium, l'hydrogénophosphate de calcium, l'hydrogéno¬phosphate de calcium dihydraté, l'acétate de calcium, l'ascorbate de calcium, le chlorure de calcium, le glucoheptonate de calcium, le glycérophosphate de calcium et/ou le sulfate de calcium.

5. Compositions pharmaceutiques contenant de l'acide zolédronique et du calcium et de la vitamine D, destinées à l'utilisation selon l'une quelconque des revendications 1-4, dans lesquelles la vitamine D comprend en particulier la vitamine D et/ou la vitamine D3 et/ou la vitamine D2 et/ou leurs dérivés, en particulier le calcitriol (1,25-dihydroxyvitamine D3) ou 1α,25-dihydroxycholécalciférol, l'alphacalcidol (1α-hydroxyvitamine D3), la 24,25-dihydroxyvitamine D3, le calcifédiol, la vitamine D2 et/ou l'ergocalciférol.

6. Compositions pharmaceutiques contenant de l'acide zolédronique et du calcium et de la vitamine D, destinées à l'utilisation selon les revendications 1-5, comprenant en outre des excipients et/ou liquides ou solvants, pharmaceutiquement acceptables.

7. Compositions pharmaceutiques contenant de l'acide zolédronique et du calcium et de la vitamine D, destinées à l'utilisation selon les revendications 1-6, dans lesquelles les excipients pour les compositions pharmaceutiques comprennent le lactose, l'amidon, des acidifiants, en particulier l'acide citrique, l'acide malique, des correcteurs d'acidité, en particulier l'hydrogénocarbonate de sodium et le carbonate de sodium, des humectants, en particulier le sorbitol, le xylitol et l'inuline, des agents de séparation, en particulier le phosphate tricalcique, des acides gras alimentaires, en particulier les sels de magnésium, notamment le stéarate de magnésium, des substances aromatiques et arômes naturels et identiques aux arômes naturels et autres, des édulcorants, en particulier le cyclamate de sodium, l'aspartame et la saccharine sodique, la maltodextrine, des colorants, en particulier la poudre de jus de betterave rouge et le riboflavine-5'-phosphate, la silice, en particulier colloïdale, la silice hydratée, la phénylalanine, la gomme arabique, le saccharose, la gélatine, l'amidon de maïs, l'huile de soja, le glycérol, le DL-alpha-tocophérol, l'isomalt, l'hydrogénocarbonate de sodium, le dihydrogénocarbonate de sodium, le citrate de sodium, le dihydrogénocitrate de sodium, la carmellose sodique, l'acésulfame potassique, l'ascorbate de sodium, des triglycérides, en particulier à longueur moyenne de chaîne, et/ou dans des liquides pharmaceutiquement acceptables, en particulier l'eau, une solution isotonique de glucose ou de chlorure de sodium.

8. Compositions pharmaceutiques contenant de l'acide zolédronique et du calcium et de la vitamine D, destinées à l'utilisation selon les revendications 1-7, dans lesquelles les liquides ou solvants pharmaceutiquement acceptables comprennent l'eau, une solution isotonique de chlorure de sodium, une solution isotonique de mannitol-acétate de sodium ou une solution isotonique de glucose.

9. Compositions pharmaceutiques contenant de l'acide zolédronique et du calcium et de la vitamine D, destinées à l'utilisation selon les revendications 1-8, dans lesquelles, individuellement ou ensemble, la composition pharmaceutique comprenant de l'acide zolédronique est fournie sous forme de solution pour perfusion, concentré de solution pour perfusion, solution pour perfusion prête à l'emploi ou seringue prête à l'emploi et les compositions pharmaceutiques comprenant du calcium et/ou de la vitamine D sous forme solide ou liquide sont fournies en tant que comprimé effervescent, comprimé ou gélule à avaler, comprimé à mâcher, granulé effervescent, granulé direct, solution buvable, gouttes, ou formulation pour pulvérisation sublinguale.
